# EUROPEAN PATENT APPLICATION

(11) **EP 4 659 671 A1**
(43) Date of publication of application: **10.12.2025**
(21) Application number: 24748817.4
(22) Date of filing: 22.01.2024
(51) Int. Cl.: A61B 5/151

(54) **BLOOD COLLECTION DEVICE**

(30) Priority: 01.02.2023 JP 2023014219
(71) Applicant: HITACHI HIGH-TECH CORPORATION, Tokyo 105-6409 (JP)
(72) Inventor: YOKOKAWA Takeshi, Tokyo 105-6409 (JP); SUGIYAMA Kimikazu, Tokyo 105-6409 (JP)
(74) Representative: Strehl & Partner mbB
(86) International application number: PCT/JP2024/001695
(87) International publication number: WO 2024/162076

(57) **Abstract**

The invention provides a blood collection device capable of reducing work involved in blood collection by adjusting an orientation around a central axis of a blood collection tube installed for blood collection and capable of stably and efficiently collecting blood into the blood collection tube. The blood collection device (1) includes a holder (110) configured to allow a blood collection tube (100) to be installed, a blood collection portion configured to collect blood from a person from whom blood is to be collected into the blood collection tube (100), and a direction adjustment mechanism (21) configured to adjust an orientation of the blood collection tube in the holder (110). The blood collection tube (100) includes a bottomed cylindrical body portion having an opening in an upper portion, and a protruding portion formed by making a part of the opening in a circumferential direction protrude upward. The direction adjustment mechanism (21) adjusts the orientation around a central axis of the blood collection tube such that a position of the protruding portion relative to the central axis of the blood collection tube (100) falls within a predetermined range set in advance.

## Description

### Technical Field

The present invention relates to a blood collection device for automatically collecting blood from a person from whom blood is to be collected.

### Background Art

In general hospitals and the like, a large number of blood collections are performed every day as pre-examination tests and the like. Medical staff members involved in blood collection work are required to perform not only blood collection but also work and business associated with blood collection, such as preparation and confirmation of a blood collection tube. The blood collection work involves many types of complicated work and tasks, which imposes a heavy burden on an operator, and thus it is desired to automate the blood collection work as much as possible.

The related art discloses a blood collection device that automatically performs blood collection. In general, a blood collection tube for collecting blood from a person from whom blood is to be collected is manually installed in a blood collection device that automatically performs blood collection. PTL 1 discloses a blood collection device attached with a module of a blood collection tube. PTL 2 discloses a blood collection tube preparation device provided with an orientation detection unit for detecting an orientation of a blood collection tube.

### Citation List

### Patent Literature

PTL 1: JP2022-109441A
PTL 2: Japanese Patent Application No. 4662193

### Summary of Invention

### Technical Problem

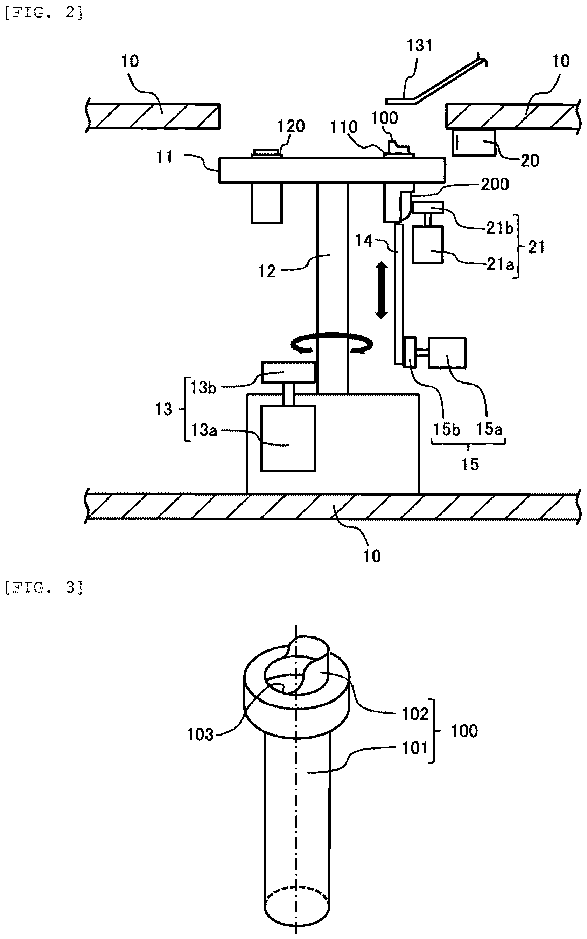

As illustrated in FIG. 3, there is a blood collection tube in which a protruding portion is formed on an opening in an upper portion. The protruding portion is formed such that a part of the opening in a circumferential direction protrudes upward. The protruding portion forms an inlet-shaped scoop around the opening. Such a protruding portion is mainly adopted in a micro blood collection tube. The protruding portion functions as a receiving surface for receiving blood and a receiving surface and a guide for an instrument such as a pipette when a fine operation is performed.

In a blood collection device that automatically performs blood collection, it is possible to efficiently perform blood collection from a blood collection site of a person from whom blood is collected to a blood collection tube by using such a protruding portion. However, in a case where blood collection is performed using the protruding portion, it is required to align an orientation of the protruding portion with a direction of the blood collection site or the like when the blood collection tube is installed in the blood collection device. It is required to adjust an orientation around a central axis of the blood collection tube so that a position of the protruding portion in a circumferential direction of an opening of the blood collection tube falls within a predetermined range relative to the central axis of the blood collection tube.

When blood collection is performed, it is required to check the type of the blood collection tubes. When blood collection is performed for a plurality of blood collection tubes, the plurality of blood collection tubes need to be installed in the blood collection device. When the blood collection tubes are installed in the blood collection device, it takes a lot of time and effort to manually adjust orientations around central axes of the blood collection tubes. Since a burden on an operator of the blood collection work increases, a time required for blood collection increases, which affects the condition of the blood and increases human errors.

Therefore, an object of the invention is to provide a blood collection device capable of reducing work involved in blood collection by adjusting an orientation around a central axis of a blood collection tube installed for blood collection and capable of stably and efficiently collecting blood into the blood collection tube.

### Solution to Problem

In order to solve the above problem, a blood collection device according to the invention includes: a holder configured to allow a blood collection tube to be installed; a blood collection portion configured to collect blood from a person from whom blood is to be collected into the blood collection tube; and a direction adjustment mechanism configured to adjust an orientation of the blood collection tube in the holder, in which the blood collection tube includes a bottomed cylindrical body portion having an opening in an upper portion, and a protruding portion formed by making a part of the opening in a circumferential direction protrude upward, and the direction adjustment mechanism adjusts the orientation around a central axis of the blood collection tube such that a position of the protruding portion relative to the central axis of the blood collection tube falls within a predetermined range set in advance.

### Advantageous Effects of Invention

According to the invention, it is possible to provide a blood collection device capable of reducing work involved in blood collection by adjusting the orientation around the central axis of the blood collection tube installed for blood collection and capable of stably and efficiently collecting blood into the blood collection tube.

### Brief Description of Drawings

[FIG. 1] FIG. 1 is an appearance view illustrating a blood collection device according to an embodiment of the invention.
[FIG. 2] FIG. 2 is a diagram schematically illustrating an example of main portions of the blood collection device according to the embodiment of the invention.
[FIG. 3] FIG. 3 is a view illustrating an example of a blood collection tube that can be used in the blood collection device according to the embodiment of the invention.
[FIG. 4] FIG. 4 is a block diagram illustrating an example of a configuration related to control of the blood collection device.
[FIG. 5] FIG. 5 is a diagram illustrating a preparation operation for electrically controlling an orientation around a central axis of the blood collection tube.
[FIG. 6] FIG. 6 is a diagram illustrating an arrangement example of a position sensor for detecting a position of a protruding portion of the blood collection tube.
[FIG. 7] FIG. 7 is a diagram illustrating an arrangement example of the position sensor for detecting a position of the protruding portion of the blood collection tube.
[FIG. 8] FIG. 8 is a diagram illustrating an arrangement example of the position sensor for detecting a position of the protruding portion of the blood collection tube.
[FIG. 9] FIG. 9 is a diagram illustrating an arrangement example of the position sensor for detecting a position of the protruding portion of the blood collection tube.
[FIG. 10] FIG. 10 is a diagram schematically illustrating an example of main portions of the blood collection device according to the embodiment of the invention.
[FIG. 11] FIG. 11 is an enlarged view illustrating the periphery of a blood collection position of the blood collection device according to the embodiment of the invention.
[FIG. 12] FIG. 12 is a top view illustrating the periphery of the blood collection position of the blood collection device according to the embodiment of the invention.
[FIG. 13] FIG. 13 is a diagram illustrating a method for mechanically adjusting an orientation around the central axis of the blood collection tube.
[FIG. 14] FIG. 14 is a diagram illustrating a method for mechanically adjusting an orientation around the central axis of the blood collection tube.
[FIG. 15] FIG. 15 is a diagram illustrating a method for mechanically adjusting an orientation around the central axis of the blood collection tube.

### Description of Embodiments

Hereinafter, a blood collection device according to an embodiment of the invention will be described. The same reference numerals are given to the same components in the following drawings, and repeated description will be omitted.

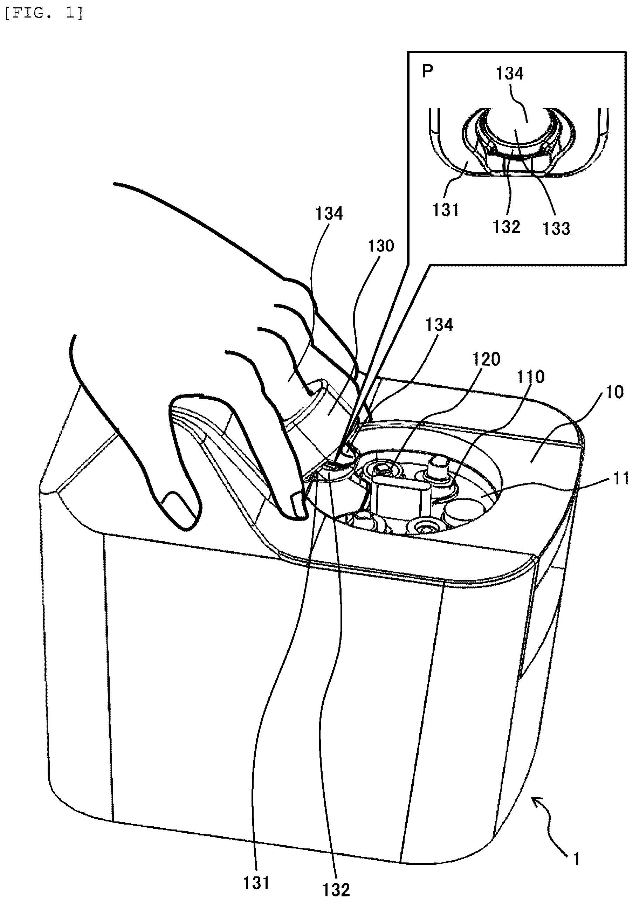

FIG. 1 is an appearance view illustrating a blood collection device according to an embodiment of the invention. FIG. 1 illustrates, as an example of a blood collection device, a finger-blood-collection device that automatically collects blood from a finger of a person from whom blood is to be collected. A reference sign P in FIG. 1 indicates an enlarged partial view of the periphery of a finger placement area of the blood collection device as viewed from below.

As illustrated in FIG. 1, the blood collection device 1 according to the present embodiment includes a housing 10, a turntable 11, a plurality of holders 110 that are installation locations of blood collection tubes, a plurality of modules 120, a cuff mechanism 130, a rotation drive mechanism (not illustrated) that rotates the turntable 11, a lifting drive mechanism (not illustrated) that lifts and lowers the holders 110 and the modules 120, a pressure adjustment mechanism (not illustrated) that drives the cuff mechanism 130, and the like.

The housing 10 is formed of a plurality of structural materials, decorative plates, and the like. The turntable 11, the rotation drive mechanism, the lifting drive mechanism, the pressure adjustment mechanism, and the like are built in the housing 10. An upper surface of the housing 10 is provided with a hand placement area where a hand of a person from whom blood is to be collected is placed, and a circular opening adjacent to the hand placement area. The turntable 11 is disposed below the opening.

The cuff mechanism 130 and a finger placement area 131 are provided on an opening side of the hand placement area. The cuff mechanism 130 is installed above the finger placement area 131 in a manner of surrounding a finger 134 of the person from whom blood is to be collected, the finger 134 being placed on the finger placement area 131. As illustrated in the partial view P, a disposable finger placement component 132 is attached to the finger placement area 131. A blood collection window 133 which is a through hole is opened in a window shape on a center side of the finger placement component 132. The finger 134 of the person from whom blood is to be collected is placed in the blood collection window 133 of the finger placement component 132.

The cuff mechanism 130 is a mechanism that tightens the periphery of the finger 134 of the person from whom blood is to be collected. A cuff is placed to surround the finger 134 placed on the finger placement area 131. For example, the cuff is provided in a flexible bag shape, and is connected to a valve or a pump via a tube. The valve and the pump constitute the pressure adjustment mechanism that drives the cuff mechanism 130. Tightening pressure on the finger 134 of the person from whom blood is to be collected is adjusted by controlling internal pressure of the cuff by the valve or the pump.

The turntable 11 is generally formed in a disk shape, and is supported inside the housing 10 such that a main surface faces upward and downward. The turntable 11 is provided with a plurality of portions that support the holders 110. Further, a plurality of holding holes that pass through the turntable 11 in an up-down direction are provided. The modules 120 are held in the holding holes in a manner of being inserted into the holding holes in an up-down direction. The portions supporting the holders 110 and the holding holes are regularly arranged at intervals along a circumferential direction of the turntable 11.

The holder 110 is an installation location for a blood collection tube, and various blood collection tubes such as a blood collection tube for a blood count test and a blood collection tube for a biochemical and immunological test are installed in the holders 110. A blood collection tube of a predetermined size or an outer tube accommodating a blood collection tube can be installed in the holder 110. The outer tube is used for the purpose of adjusting a size of an installation object relative to an installation location of the blood collection tube.

The module 120 is detachably attached to the turntable 11. A flange-shaped portion having a diameter larger than an inner diameter of the holding hole of the turntable 11 is formed on the module 120. The module 120 is inserted into the holding hole and the flange-shaped portion is supported from below, so that the module 120 is held in the holding hole in a state where the module 120 can be moved up and down.

For example, different types of modules such as a puncture module and a hemostatic module can be mounted as the modules 120. A puncture device is attached to the puncture module. A puncture needle (lancet) is built in the puncture device. A gauze or an adhesive plaster is attached to the hemostatic module.

When the puncture device is pressed against a finger or the like of a person from whom blood is to be collected, the puncture needle protrudes to puncture the finger of the person from whom blood is to be collected. The gauze is pressed against a blood collection site of the person from whom blood is collected, and absorbs blood coming out from the blood collection site to perform hemostasis. The adhesive plaster is pressed against and attached to the blood collection site of the person from whom blood is collected, and seals the blood collection site or performs hemostasis. The adhesive plaster is attached to the module such that an adhesive surface faces upward.

FIG. 2 is a diagram schematically illustrating an example of main portions of the blood collection device according to the embodiment of the invention. FIG. 2 schematically illustrates an example of structures on the periphery of the turntable 11 installed inside the blood collection device 1.

As illustrated in FIG. 2, the turntable 11, a shaft 12, a rotation drive mechanism 13, a movable support member 14, a lifting drive mechanism 15, and the like are built in the blood collection device 1 below the opening of the housing 10. The rotation drive mechanism 13, the movable support member 14, and the lifting drive mechanism 15 are supported at predetermined positions by support members (not illustrated).

The shaft 12 is coupled to the center of the turntable 11. The other end of the shaft 12 is rotatably supported by the rotation drive mechanism 13. The rotation drive mechanism 13 includes a motor 13a and a power transmission mechanism 13b. The power transmission mechanism 13b couples an output shaft of the motor 13a and the shaft 12 via a predetermined mechanical mechanism. A rotational motion of the motor 13a is transmitted to the shaft 12 by the power transmission mechanism 13b. The rotation drive mechanism 13 rotates the turntable 11 by such a mechanism.

The turntable 11 can be rotated by the rotation drive mechanism 13 in both clockwise and counterclockwise directions about a rotation axis passing through the center of a main surface. Rotation of the turntable 11 by a predetermined step angle is controlled according to a blood collection operation or a treatment operation. The rotation of the turntable 11 enables each of the holders 110 and the modules 120 to be conveyed to a blood collection position where the finger placement area 131 is formed.

At the blood collection position, puncturing with a puncture needle using a puncture module, hemostasis with a gauze using a hemostatic module, and attachment of an adhesive plaster using a hemostatic module are performed in this order. After the finger 134 of the person from whom blood is to be collected is pressed by the cuff mechanism 130, the puncture needle punctures the finger 134 using the puncture module. Blood flowing out from the blood collection site is collected into the blood collection tube that was moved to the blood collection position on the turntable 11.

The movable support member 14 is disposed below the blood collection position. The movable support member 14 is supported by the lifting drive mechanism 15 in a manner that the movable support member 14 can be freely moved up and down. The lifting drive mechanism 15 includes a motor 15a and a power transmission mechanism 15b. The power transmission mechanism 15b couples an output shaft of the motor 15a and the movable support member 14 via a predetermined mechanical mechanism. A rotational motion of the motor 15a is converted into an up-down linear motion by the power transmission mechanism 15b. The lifting drive mechanism 15 moves the movable support member 14 up and down by such a mechanism.

When the holder 110 or the module 120 is moved to the blood collection position by the rotation of the turntable 11, the holder 110 or the module 120 is driven to move up and down relative to the finger placement area 131 by being pushed upward from a lower side by an upward movement of the movable support member 14 and being lowered downward to a lower side by a downward movement of the movable support member 14. By such an operation, the blood collection tube is pressed against the blood collection site, the puncture needle is inserted into the finger 134 of the person from whom blood is to be collected, and a gauze or an adhesive plaster is pressed and attached to the blood collection site.

FIG. 3 is a diagram illustrating an example of a blood collection tube that can be used in the blood collection device according to the embodiment of the invention.

As illustrated in FIG. 3, as a blood collection tube for collecting blood from a person from whom blood is to be collected, there is a blood collection tube 100 in which a protruding portion 102 is formed at an opening in an upper portion. The blood collection tube 100 having the protruding portion 102 as illustrated in FIG. 3 can be used in the blood collection device 1.

The blood collection tube 100 illustrated in FIG. 3 includes a bottomed cylindrical body portion 101 having an opening in an upper portion, and the protruding portion 102 protruding upward. An opening 103 is formed in an upper portion of the body portion 101. The opening 103 has a circular shape in a top view of the blood collection tube 100 and is coupled to the inside of the body portion 101. The protruding portion 102 is provided in an upper portion of the body portion 101, and a part of the opening 103 in a circumferential direction protrudes upward from the body portion 101.

The protruding portion 102 has a surface continuous from an inner peripheral surface of the body portion 101, and is curved with the same curvature as the inner peripheral surface of the body portion 101. The protruding portion 102 forms an inlet-shaped scoop around the opening 103. When the blood collection tube 100 is inclined such that the protruding portion 102 is on a lower side, the protruding portion 102 has an inlet shape that easily receives liquid droplets or the like. The protruding portion 102 may function as a receiving surface for receiving blood collected into the blood collection tube 100 and a receiving surface and a guide for an instrument such as a pipette.

When the blood collection tube 100 as illustrated in FIG. 3 is used in the blood collection device 1, it is desirable to align an orientation of the protruding portion 102 with a direction of the blood collection site of the person from whom blood is to be collected. From the viewpoint of reducing the burden on an operator in the blood collection work or from the viewpoint of improving collection performance to collect the blood into the blood collection tube 100, even when the blood collection tube 100 is installed in the holder 110 in any orientation, it is preferable that the protruding portion 102 faces a predetermined orientation when the blood collection tube 100 is moved to the blood collection position by the rotation of the turntable 11.

Therefore, in the blood collection device 1 according to the present embodiment, control is performed to adjust the orientation around the central axis of the blood collection tube 100 so that a position of the protruding portion 102 relative to the central axis (one-dot chain line in FIG. 3) of the blood collection tube 100 falls within a predetermined range set in advance. By adjusting the orientation around the central axis of the blood collection tube 100, the position of the protruding portion 102 relative to the central axis of the blood collection tube 100 in the circumferential direction of the opening 103, that is, the orientation of the protruding portion 102 relative to the central axis is adjusted.

The orientation around the central axis of the blood collection tube 100 is adjusted such that the relative position of the protruding portion 102 at the blood collection position on the turntable 11 falls within a target range. The orientation around the central axis of the blood collection tube 100 is adjusted in a preparation operation performed before the start of a blood collection operation. The blood collection operation is an operation of puncturing the person from whom blood is to be collected with a puncture needle and collecting blood from the person from whom blood is collected into the blood collection tube.

The orientation around the central axis of the blood collection tube 100 can be adjusted to any orientation. The position of the protruding portion 102 relative to the central axis of the blood collection tube 100 can be adjusted to fall within any target range. The target range can be set in advance when the blood collection device 1 is used. The target range can have any range width according to a lateral width of the protruding portion 102 or the like.

The target range of the position of the protruding portion 102 relative to the central axis of the blood collection tube 100 can be set as a partial annular region or the like having any arc length along the circumferential direction of the opening 103 of the blood collection tube 100 in the top view of the blood collection tube 100 that was moved to the blood collection position on the turntable 11. The orientation around the central axis of the blood collection tube 100 is adjusted such that both side ends of the protruding portion 102 fall in such a region.

The target range of the position of the protruding portion 102 relative to the central axis of the blood collection tube 100 is preferably set to a half region located closer to a center side of the turntable 11 than the central axis of the blood collection tube 100 or a half region located closer to an outer side of the turntable 11 than the central axis of the blood collection tube 100. That is, relative to the central axis of the blood collection tube 100, it is preferable to set the target range in a half region close to the blood collection site of the person from whom blood is to be collected or a half region far from the blood collection site of the person from whom blood is to be collected.

For example, the blood collection device 1 may have a function of inclining the blood collection tube moved to the blood collection position along a radial direction of the turntable 11. In such a case, the orientation of the protruding portion 102 at the blood collection position is preferably a forward direction or a reverse direction relative to an inclination orientation of the blood collection tube from the viewpoint of arranging the inlet shape. When the target range is a half region located on the center side or the outer side of the turntable 11, blood can be efficiently collected along the protruding portion 102.

As illustrated in FIG. 2, the blood collection tube 100 is installed in the holder 110. In FIG. 2, the blood collection tube 100 is installed in the holder 110 in a state where the blood collection tube 100 is accommodated in an outer tube 200. In such a case, the position of the protruding portion 102 relative to the central axis of the blood collection tube 100 can be adjusted by changing an orientation around a central axis of the outer tube 200.

In FIG. 2, the holder 110 is provided in a shape in which a part of the outer peripheral surface on an outer side is opened. The holder 110 is provided in a structure that opens a side surface of an installation object toward the outside. With such a structure, an orientation of the installation object installed in the holder 110 can be adjusted from the outside. The holder 110 can be provided in an appropriate structure according to a method for adjusting the orientation around the central axis of the blood collection tube 100.

In FIG. 2, a position sensor 20 is installed on a side of the blood collection position. The position sensor 20 is a sensor for detecting a position of the protruding portion 102 of the blood collection tube 100. The position sensor 20 detects the position of the protruding portion 102, for example, a position of a side end of the protruding portion 102 of the blood collection tube 100 that is moved to the blood collection position on the turntable 11. The orientation around the central axis of the blood collection tube 100 can be controlled based on a detection result of the position sensor 20.

An optical sensor, a camera type sensor, or the like can be used as the position sensor 20. The position sensor 20 can be installed at a fixed point such as a side of the protruding portion 102 so as to monitor a region of the target range of the position of the protruding portion 102 relative to the central axis of the blood collection tube 100 at the blood collection position on the turntable 11. As the position sensor 20, one sensor may be installed, or a plurality of sensors may be installed.

The optical sensor includes a light source (light projecting unit) that emits laser light or the like and a light sensor (light receiving unit) that detects reflected light or scattered light of the emitted light. When light such as laser light is emitted to a region in the target range of the position of the protruding portion 102 relative to the central axis of the blood collection tube 100, which is the height of the protruding portion 102 at the blood collection position, and a light intensity of reflected light or scattered light is measured, a position of a side end of the protruding portion 102 can be detected. The light source and the light sensor constituting the optical sensor may be integrally provided at the same place or may be separately provided at different places.

The camera type sensor images the periphery of the region of the target range of the position of the protruding portion 102 relative to the central axis of the blood collection tube 100, which is the height of the protruding portion 102 at the blood collection position. The position of the protruding portion 102 can be detected by performing image processing on the imaged image. The camera-type sensor may employ a plurality of cameras or a stereo camera.

From the viewpoint of ensuring an installation location and convenience of detection processing, an optical sensor is preferably used or a reflective optical sensor is preferably used as the position sensor 20. In the case of an optical sensor, the position of the protruding portion 102 relative to the central axis of the blood collection tube 100 in the circumferential direction of the opening 103 can be obtained by detecting passage of the side end of the protruding portion 102 through the target range.

In FIG. 2, an electric direction adjustment mechanism 21 that adjusts an orientation of the blood collection tube 100 in the holder 110 is installed below the blood collection position. The direction adjustment mechanism 21 includes a motor 21a and a direction adjustment unit 21b. The direction adjustment unit 21b is coupled to an output shaft of the motor 21a. The motor 21a rotates the direction adjustment unit 21b at a predetermined step angle. The direction control mechanism 21 operates the direction adjustment unit 21b by the motor 21a to rotate an installation object installed in the holder 110.

The motor 21a may be implemented by a stepping motor, a servo motor, or the like. The direction adjustment unit 21b is disposed in a manner of coming into contact with an outer peripheral surface of the installation object installed in the holder 110 which is a direction adjustment target. The direction adjustment unit 21b can be formed of a material having large friction resistance with respect to the installation object installed in the holder 110. By operating the direction adjustment unit 21b using the motor 21a, an orientation around a central axis of the installation object installed in the holder 110 can be changed due to the frictional force with the direction adjustment unit 21b.

For example, a roller-shaped pad formed of a material having large friction resistance can be used as the direction adjustment unit 21b. By rotating the roller-shaped pad using the motor 21a, the orientation around the central axis of the installation object installed in the holder 110 can be changed by the frictional force caused by the rotation of the roller-shaped pad.

In addition to the roller-shaped pad, a belt-shaped pad formed of a material having large friction resistance, a plate-shaped pad formed of a material having large friction resistance, or the like may be used as the direction adjustment unit 21b. In addition to a method for pressing the pad against a side surface of the installation object, an arm or the like can be used. The direction adjustment mechanism 21 can be driven by an appropriate mechanical mechanism according to a type of the direction adjustment unit 21b, a structure on the periphery of the blood collection tube 100, and the like.

An appropriate material can be used as the material of the direction adjustment unit 21b as long as friction resistance received by a surface of the direction adjustment unit 21b that came into contact with the installation object installed in the holder 110 is larger than friction resistance received by a surface of the holder 110 that came into contact with the installation object. Examples of the material of the direction adjustment unit 21b include elastomer such as rubber, a porous material such as sponge, and fabric provided with napped surface.

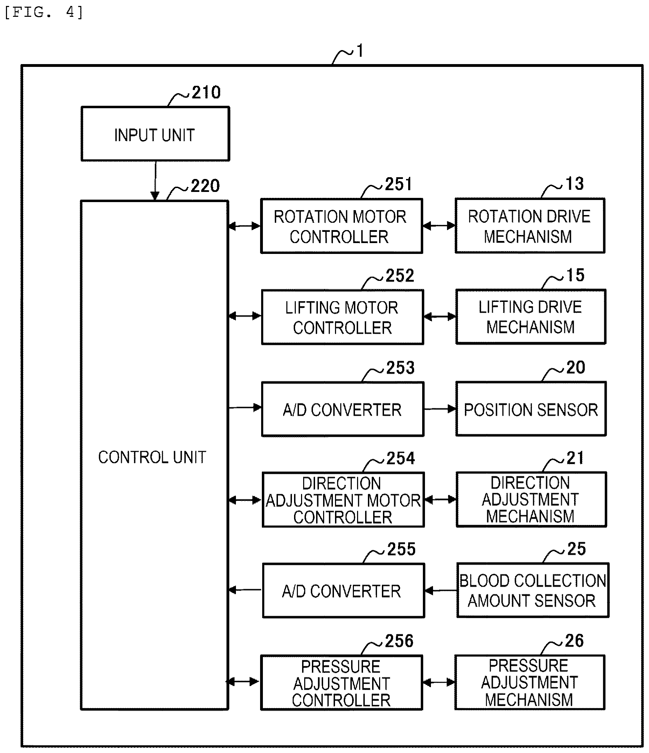

FIG. 4 is a block diagram illustrating an example of a configuration related to control of the blood collection device. FIG. 4 illustrates a configuration related to control of main components related to an operation of the blood collection device 1.

As illustrated in FIG. 4, the blood collection device 1 includes an input unit 210, a control unit 220, a storage unit (not illustrated) that stores programs and data, a display unit that displays information related to blood collection, and the like. The direction control mechanism 21 can be controlled by the control unit 220 according to a predetermined program.

The input unit 210 receives an input from a user of the blood collection device 1. The input unit 210 is implemented by, for example, a touch panel and various switches. Various kinds of data, instructions, and the like can be input to the blood collection device 1 by the input unit 210 or an external device.

The control unit 220 executes processing according to a program, reads a program or data, and controls main components related to an operation of the blood collection device 1. The control unit 220 includes, for example, a calculation device such as a central processing unit (CPU) and a storage device such as a random access memory (RAM) and a read only memory (ROM).

The blood collection device 1 includes, as main components related to the operation of the blood collection device 1, the rotation drive mechanism 13 that rotates the turntable 11, the lifting drive mechanism 15 that lifts and lowers the holders 110 and the modules 120, the position sensor 20, the direction adjustment mechanism 21, a blood collection amount sensor 25, a pressure adjustment mechanism 26 that drives the cuff mechanism 130, and the like. These components are controlled by the control unit 220.

The rotation drive mechanism 13 is connected to the control unit 220 via a motor controller 251 so that signal communication can be performed. The lifting drive mechanism 15 is connected to the control unit 220 via a motor controller 252 so that signal communication can be performed. The position sensor 20 is connected to the control unit 220 via an A/D converter 253 so that signal communication can be performed.

The direction control mechanism 21 is connected to the control unit 220 via a motor controller 254 so that signal communication can be performed. The blood collection amount sensor 25 is connected to the control unit 220 via an A/D converter 255 so that signal communication can be performed. The pressure adjustment mechanism 26 is connected to the control unit 220 via a pressure adjustment controller 256 so that signal communication can be performed.

The blood collection amount sensor 25 is a sensor for measuring a blood collection amount from a person from whom blood is to be collected into a blood collection tube. The blood collection amount sensor 25 is installed on a side of a blood collection tube installed on the turntable 11. The blood collection amount sensor 25 can be implemented by a light source that emits light toward the blood collection tube and a light sensor that detects reflected light or scattered light of the emitted light.

The blood collection amount sensor 25 measures a light intensity of reflected light or scattered light from the blood collected in the blood collection tube, and measures a height of a liquid surface of the blood collected in the blood collection tube. The blood collection amount of blood collected into the blood collection tube can be obtained by calculation based on a measurement result of the height of the liquid surface of the blood collected in the blood collection tube, an inner diameter of the blood collection tube, and the like.

The pressure adjustment mechanism 26 is implemented by a valve or a pump connected to the cuff of the cuff mechanism 130. Internal pressure of the cuff is controlled by opening and closing the valve or adjusting the pressure by the pump to control tightening pressure on the finger 134 of the person from whom blood is to be collected. The pressure adjustment mechanism 26 may be built in the housing 10 of the blood collection device 1.

The control unit 220 can be provided as a system control unit, and sets target values for controlling the rotation drive mechanism 13, the lifting drive mechanism 15, the direction adjustment mechanism 21, and the pressure adjustment mechanism 26 according to programs or detection results of various sensors. The control unit 220 performs sequence control among the components. A control signal for a target value is transmitted from the control unit 220 to the motor controllers 251, 252, 254 and the pressure adjustment controller 256.

The rotation drive mechanism 13, the lifting drive mechanism 15, the direction adjustment mechanism 21, and the pressure adjustment mechanism 26 are respectively controlled by the motor controllers 251, 252, 254 and the pressure adjustment controller 256, so as to operate with set target control amounts according to a blood collection operation or a treatment operation. These components may be configured to sense a control amount as necessary. The control amount can be feedback-controlled by sensing the control amount.

The position sensor 20 detects a position of the protruding portion 102 of the blood collection tube 100 installed in the holder 110, and inputs an analog detection signal indicating a detection result to the A/D converter 253. The A/D converter 253 converts the analog detection signal into a digital detection signal. The digital detection signal is amplified as necessary and then input to the control unit 220. The same processing is performed in the blood collection amount sensor 25 and the A/D converter 255.

The control unit 220 compares the detection result of the position of the protruding portion 102 detected by the position sensor 20 with a preset target range. The control unit 220 can read data indicating the target range from a storage device, compare the data with the detection result of the position sensor 20, and obtain a deviation between a detected current position and a preset target position. A control amount for changing the orientation around the central axis of the blood collection tube 100 can be set based on such a deviation.

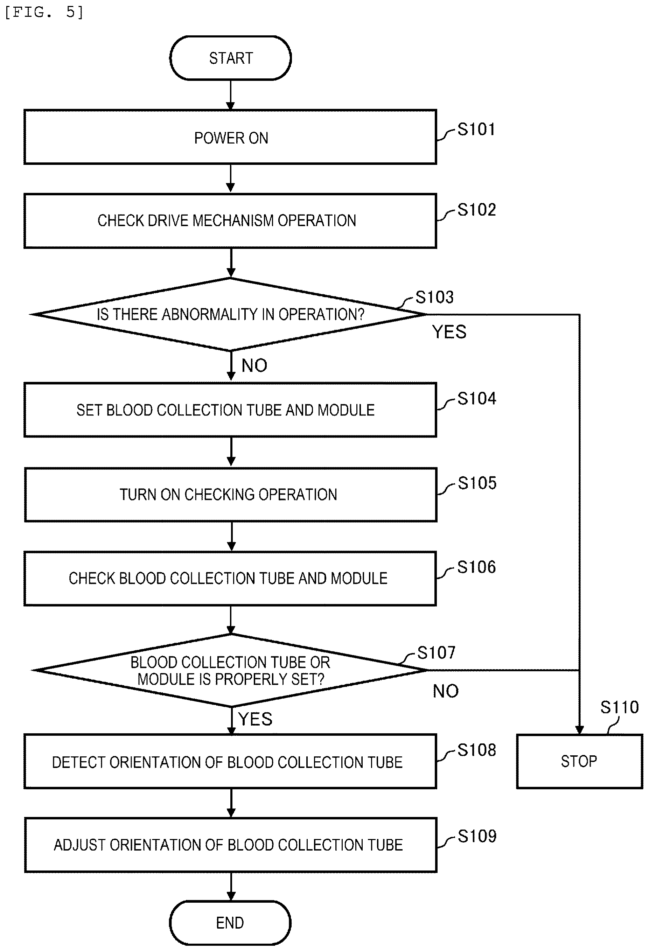

FIG. 5 is a diagram illustrating a preparation operation for electrically adjusting the orientation around the central axis of the blood collection tube. FIG. 5 illustrates a flow of a preparation operation performed when the blood collection device 1 is started in a case where the orientation around the central axis of the blood collection tube 100 is adjusted by the electric direction adjustment mechanism 21.

As illustrated in FIG. 5, the detection of the orientation around the central axis of the blood collection tube 100, that is, the detection of the position of the protruding portion 102 of the blood collection tube 100 and the adjustment of the orientation around the central axis of the blood collection tube 100 can be performed when the blood collection device 1 is started.

Data indicating a target range of the position of the protruding portion 102 relative to the central axis of the blood collection tube 100 can be stored in a storage unit of the blood collection device 1 in advance before blood collection. For example, when an optical sensor is used as the position sensor 20, a threshold of a light intensity for identifying the presence or absence of the protruding portion 102 in a monitoring region can be prepared as the data indicating the target range of the relative position of the protruding portion 102. When a camera type sensor is used as the position sensor 20, a reference image or the like for identifying the presence or absence of the protruding portion 102 in a monitoring region can be prepared.

When blood collection is performed using the blood collection device 1, first, power of the blood collection device 1 is turned on (step S101). A user of the blood collection device 1 starts the blood collection device 1 by pressing a power button or the like.

Subsequently, operations of drive mechanisms such as the rotation drive mechanism 13, the lifting drive mechanism 15, and the direction adjustment mechanism 21 are checked (step S102). When there is an abnormality in the operation of the drive mechanism (step S103: NO), the preparation operation is stopped. On the other hand, when there is no abnormality in the operation of the drive mechanism (step S103: YES), the processing proceeds to step S104.

Subsequently, a blood collection tube or a module is installed on the turntable 11 of the blood collection device 1 (step S104). The user of the blood collection device 1 checks that there is no abnormality in the operation of the drive mechanism by display or the like, and installs a predetermined type of blood collection tube 100 used for blood collection in the holder 110. In addition, a puncture module or a hemostatic module is installed in the holding hole of the turntable 11.

Subsequently, a checking operation of the blood collection device 1 is turned on (step S105). After installing the blood collection tube 100 or the module, the user of the blood collection device 1 presses a predetermined button or the like to start the checking operation of the blood collection tube 100 or the module installed in the holder 110.

Subsequently, the blood collection tube 100 or the module is checked (step S106). When the blood collection tube 100 or the module is not properly installed (step S107: NO), the preparation operation is stopped. On the other hand, when the blood collection tube 100 or the module is properly installed (step S107: YES), the processing proceeds to step S108.

Subsequently, the orientation around the central axis of the blood collection tube 100 is detected (step S108). The position of the protruding portion 102 relative to the central axis of the blood collection tube 100 in the circumferential direction of the opening 103 is detected by the position sensor 20 installed on a side of the protruding portion 102 of the blood collection tube 100. When a plurality of blood collection tubes 100 are installed on the turntable 11, the turntable 11 can be rotated to sequentially detect the blood collection tubes 100.

Subsequently, the orientation around the central axis of the blood collection tube 100 is adjusted (step S109). The orientation around the central axis of the blood collection tube 100 is adjusted by the electric direction adjustment mechanism 21 installed below the blood collection position such that the position of the protruding portion 102 relative to the central axis of the blood collection tube 100 falls within a preset target range. When a plurality of blood collection tubes 100 are installed on the turntable 11, the adjustment can be performed for each detection.

After the orientation around the central axis of the blood collection tube 100 is adjusted, a blood collection condition can be input and read. After a blood collection condition is set as necessary, the finger 134 of the person from whom blood is to be collected can be set in the finger placement area 131, the finger 134 of the person from whom blood is to be collected can be punctured with a puncture needle, blood can be collected into the blood collection tube 100, and hemostasis at the blood collection site can be performed. The blood collection condition includes a time from the puncture with the puncture needle, a time of pressing and releasing by the cuff mechanism 130, the number of times of repetitions, and the like.

According to such a blood collection device, since the position sensor 20 and the electric direction adjustment mechanism 21 are provided, the orientation around the central axis of the blood collection tube 100 can be accurately and actively controlled based on a detection result of a sensor. The position of the protruding portion 102 relative to the central axis of the blood collection tube 100 can be adjusted within a predetermined target range based on a current position detected by the sensor. In addition, it is not necessary for an operator who performs the blood collection work to check the orientation of the protruding portion 102, and the protruding portion 102 subjected to orientation adjustment can be used as a blood receiving surface or a receiving surface and a guide of an instrument. Therefore, the orientation around the central axis of the blood collection tube installed for blood collection can be adjusted by accurate and reliable automatic control, work related to blood collection can be reduced, and efficient blood collection into the blood collection tube can be stably performed.

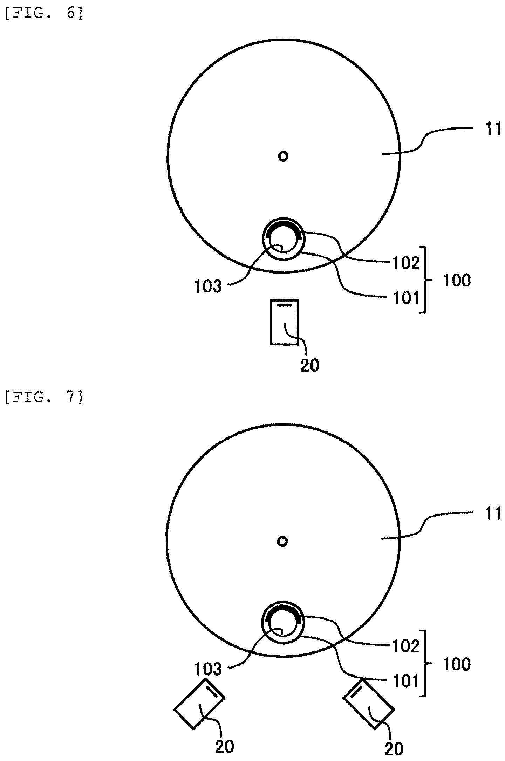

FIGS. 6, 7, 8, and 9 are diagrams illustrating arrangement examples of a position sensor for detecting the position of the protruding portion of the blood collection tube. FIGS. 6, 7, 8, and 9 schematically illustrate the arrangement examples of the position sensor 20 when the turntable 11 built in the blood collection device 1 is viewed from above. In these drawings, a detailed structure of the turntable 11, the holder 110, and the outer tube 200 are not illustrated.

FIGS. 6, 7, 8 and 9 illustrate a state where the blood collection tube 100 is stopped at the blood collection position on the turntable 11. The position of the protruding portion 102 relative to the central axis of the blood collection tube 100 is adjusted within a predetermined target stop range. The target stop range is set in a half region located closer to a center side of the turntable 11 than the central axis of the blood collection tube 100.

As illustrated in FIG. 6, the position sensor 20 can be installed outside the turntable 11 in a top view of the turntable 11. According to such a form, at the height of the protruding portion 102 of the blood collection tube 100 stopped at the blood collection position, movement of a side end of the protruding portion 102 relative to the target stop range can be detected by one sensor. Since detection can be performed by one sensor outside the turntable 11, installation and wiring of the sensor can be facilitated.

As illustrated in FIG. 7, the position sensor 20 may be installed as a combination of a plurality of sensors outside the turntable 11 in a top view of the turntable 11. According to such a form, at the height of the protruding portion 102 of the blood collection tube 100 stopped at the blood collection position, movement of a side end of the protruding portion 102 relative to the target stop range can be individually detected by the plurality of sensors. Since detection can be performed outside the turntable 11, installation and wiring of the sensors can be facilitated. In addition, since a plurality of positions can be monitored by a plurality of sensors, processing of obtaining the position of the protruding portion 102 is facilitated.

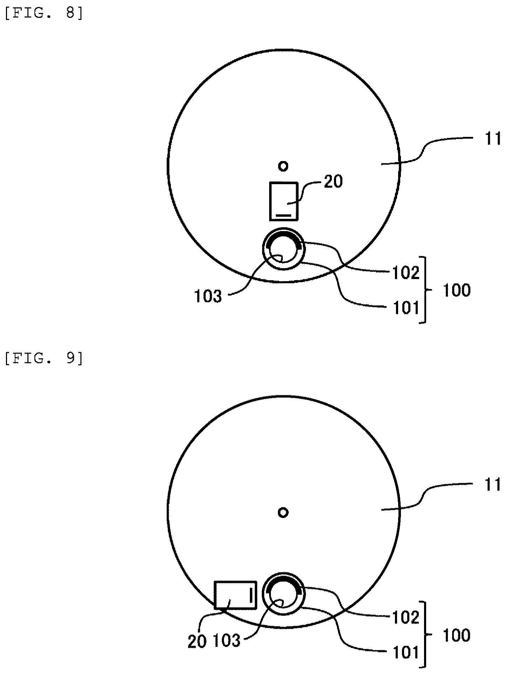

As illustrated in FIG. 8, the position sensor 20 may be installed on the turntable 11 and closer to the center side of the turntable 11 than the blood collection tube 100. According to such a form, movement of a side end of the protruding portion 102 relative to the target stop range can be detected at the height of the protruding portion 102 on the turntable 11. In such a form, wiring through the shaft 12, flexible printed circuits (FPC) related to the sensor, and the like are required, and a torsion release operation or the like for a rotation operation is required, and installation and wiring of the sensor are difficult. However, since the position of the position sensor 20 relative to the blood collection tube 100 is fixed, monitoring can be performed without being affected by operation accuracy of the turntable 11 before the blood collection tube 100 is moved to the blood collection position.

As illustrated in FIG. 9, the position sensor 20 may be installed on the turntable 11 and on a side of the blood collection tube 100 along the circumferential direction of the turntable 11. According to such a form, movement of a side end of the protruding portion 102 relative to the target stop range can be detected at the height of the protruding portion 102 on the turntable 11. In such a form, installation and wiring of the sensor is difficult in a similar manner to the form in FIG. 8. However, since the position of the position sensor 20 relative to the blood collection tube 100 is fixed, monitoring can be performed without being affected by operation accuracy of the turntable 11 before the blood collection tube 100 is moved to the blood collection position.

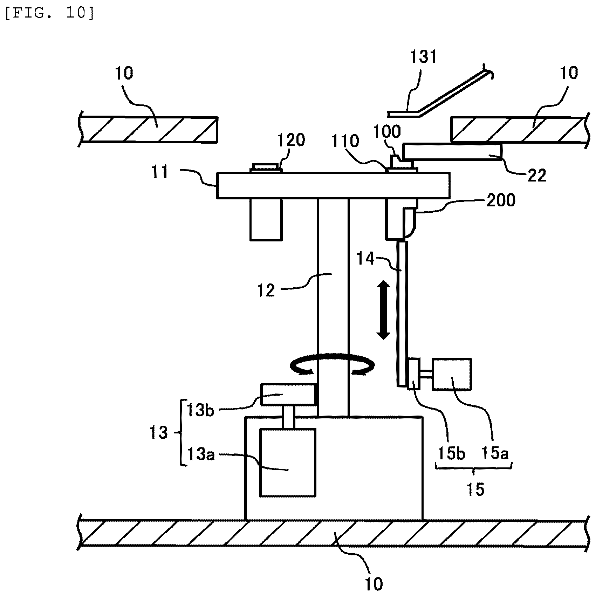

FIG. 10 is a diagram schematically illustrating an example of main portions of the blood collection device according to the embodiment of the invention. FIG. 10 schematically illustrates another example of a structure on the periphery of the turntable 11 installed inside the blood collection device 1.

As illustrated in FIG. 10, the turntable 11, the shaft 12, the rotation drive mechanism 13, the movable support member 14, the lifting drive mechanism 15, and the like are incorporated in the blood collection device 1 below the opening of the housing 10. The rotation drive mechanism 13, the movable support member 14, and the lifting drive mechanism 15 are supported at predetermined positions by support members (not illustrated).

The blood collection device 1 illustrated in FIG. 10 is different from the blood collection device 1 illustrated in FIG. 2 in that a mechanical direction adjustment mechanism 22 is provided instead of the position sensor 20 and the electric direction adjustment mechanism 21. As illustrated in FIG. 10, the mechanical direction adjustment mechanism 22 can also be used as an adjustment mechanism for adjusting the orientation of the blood collection tube 100 in the holder 110.

As illustrated in FIG. 10, the blood collection tube 100 is installed in the holder 110. In FIG. 10, the blood collection tube 100 is installed in the holder 110 in a state where the blood collection tube 100 is accommodated in the outer tube 200. The holder 110 is provided in a shape in which a part of the outer peripheral surface on an outer side is opened. However, the holder 110 can be provided in an appropriate structure as long as the structure does not fix the orientation around the central axis of the installation object.

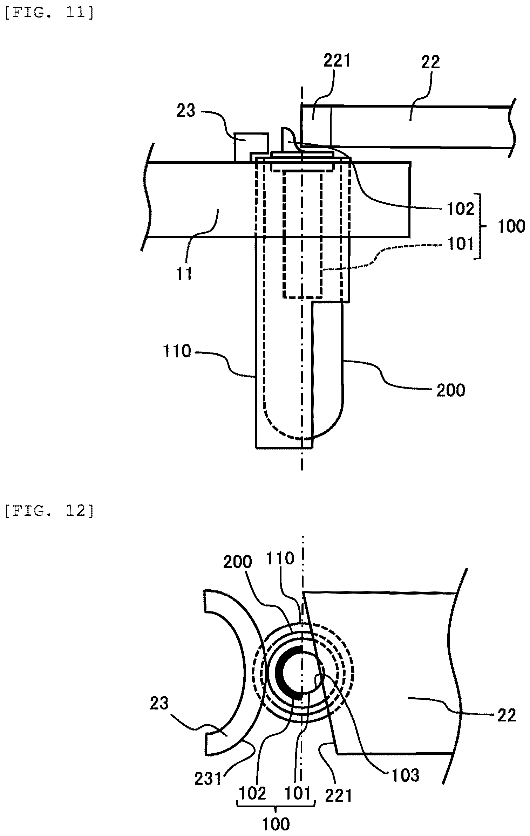

In FIG. 10, the mechanical direction adjustment mechanism 22 for adjusting the orientation of the blood collection tube 100 in the holder 110 is installed on a side of a blood collection position. The direction adjustment mechanism 22 includes a guide that moves the protruding portion 102 of the blood collection tube 100 to a predetermined position. As illustrated in FIG. 11, the direction adjustment mechanism 22 has an inclined guide surface 221 that guides the movement of the protruding portion 102.

The direction adjustment mechanism 22 is disposed such that the guide surface 221 provided on a tip end side extends along the vicinity of the target stop range of the protruding portion 102 at the blood collection position on the turntable 11. The direction adjustment mechanism 22 can be attached to, for example, an inner surface side of the housing 10 above the turntable 11. Alternatively, the direction adjustment mechanism 22 can be attached to an appropriate position as long as a position of the guide surface 221 relative to the target stop range of the protruding portion 102 is fixed.

The direction adjustment mechanism 22 may be formed of an appropriate material. Examples of the material of the direction adjustment mechanism 22 include elastomer, plastic, and metal. From the viewpoint of reducing impact on the protruding portion 102, or the like, an elastomer such as rubber is preferably used as the material of the direction adjustment mechanism 22.

FIG. 11 is an enlarged view illustrating the periphery of a blood collection position of the blood collection device according to the embodiment of the invention. FIG. 12 is a top view illustrating the periphery of the blood collection position of the blood collection device according to the embodiment of the invention. FIG. 11 illustrates the periphery of the blood collection position where the direction adjustment mechanism 22 illustrated in FIG. 10 is installed. FIG. 12 illustrates the periphery of the blood collection position illustrated in FIG. 11 as viewed from above.

FIGS. 11 and 12 illustrate a state where the blood collection tube 100 is stopped at a blood collection position on the turntable 11. The position of the protruding portion 102 relative to the central axis of the blood collection tube 100 is adjusted within a predetermined target stop range. The target stop range is set in a half region located closer to the center side of the turntable 11 than the central axis of the blood collection tube 100. Such adjustment to the target stop range is performed by the direction adjustment mechanism 22.

As illustrated in FIGS. 11 and 12, the blood collection tube 100 can be installed in the holder 110 in a state where the blood collection tube 100 is accommodated in the outer tube 200. The protruding portion 102 of the blood collection tube 100 protrudes upward from the body portion 101, the outer tube 200, or the holder 110. A height relationship among the blood collection tube 100, the outer tube 200, and the holder 110 on the turntable 11 can be appropriately set as long as the protruding portion 102 protrudes upward.

As illustrated in FIG. 11, the guide surface 221 of the direction adjustment mechanism 22 is provided above the turntable 11 at a height at which the guide surface 221 can come into contact with the protruding portion 102 of the blood collection tube 100 and at a height at which the guide surface 221 does not come into contact with the body portion 101 of the blood collection tube 100, the outer tube 200, and the holder 110. When the position of the protruding portion 102 relative to the central axis of the blood collection tube 100 is adjusted toward the center side of the turntable 11, the direction adjustment mechanism 22 is provided closer to the outside of the turntable 11 than the protruding portion 102.

As illustrated in FIG. 12, the guide surface 221 of the direction adjustment mechanism 22 is provided inclined relative to a tangential axis (two-dot chain line in FIG. 12) that passes through the center of the blood collection tube 100 and is parallel to the tangential line of the turntable 11 in a top view of the turntable 11. The guide surface 221 is provided at a position where the guide surface 221 interferes with the protruding portion 102 outside the target stop range and does not interfere with the protruding portion 102 within the target stop range when the blood collection tube 100 is moved to the blood collection position by the rotation of the turntable 11.

The guide surface 221 can be provided in a manner of intersecting the opening 103 in a top view of the blood collection tube 100 stopped at the blood collection position on the turntable 11. According to such a structure, when the blood collection tube 100 is moved to the blood collection position by the rotation of the turntable 11, the protruding portion 102 can be brought into contact with the guide surface 221 regardless of the position of the protruding portion 102 relative to the central axis of the blood collection tube 100. Therefore, the orientation of the protruding portion 102 relative to the central axis of the blood collection tube 100 can be changed by preventing a translational motion of the protruding portion 102 by the guide surface 221.

One end of the guide surface 221 located on an inlet side of the protruding portion 102 is separated from the tangential axis. On the other hand, the other end of the guide surface 221 located on an exit side of the protruding portion 102 is close to the tangential axis. The other end side of the guide surface 221 and a side end of the protruding portion 102 in the target stop range can be provided in a manner of being close to each other in a top view of the blood collection tube 100 stopped at the blood collection position on the turntable 11. According to such a structure, the blood collection tube 100 can be rotated around the central axis to stop the side end of the protruding portion 102 in the vicinity of the target stop range.

As illustrated in FIGS. 11 and 12, a back surface support member 23 can be provided on the turntable 11. The back surface support member 23 is provided on a side opposite to the direction adjustment mechanism 22 across the blood collection tube 100. The back surface support member 23 is a member for supporting the blood collection tube 100 from a side of a back surface side when the protruding portion 102 comes into contact with the direction adjustment mechanism 22. When the protruding portion 102 comes into contact with the direction adjustment mechanism 22, the rotation of the blood collection tube 100 around the central axis can be smoothed by supporting the blood collection tube 100 from the side of the back surface side.

The back surface support member 23 can be provided on the turntable 11 at a height at which the back surface support member 23 comes into contact with the body portion 101 of the blood collection tube 100 or the outer tube 200 that accommodates the blood collection tube 100 which is a direction adjustment target, and at a height at which the back surface support member 23 does not come into contact with the protruding portion 102 of the blood collection tube 100. When the position of the protruding portion 102 relative to the central axis of the blood collection tube 100 is adjusted toward the center side of the turntable 11, the back surface support member 23 is provided closer to the center side of the turntable 11 than the protruding portion 102.

The back surface support member 23 has a support surface 231 that supports an installation object installed in the holder 110 that is a direction control target, for example, supports the body portion 101 of the blood collection tube 100 or the outer tube 200 that accommodates the blood collection tube 100. The support surface 231 is disposed along a back surface side of the protruding portion 102 in the target stop range so as to face the guide surface 221 across the body portion 101 of the blood collection tube 100 or the outer tube 200 that accommodates the blood collection tube 100.

In FIG. 12, the support surface 231 is provided in a curved shape protruding toward the outside of the turntable 11. With such a shape, the support surface 231 can be brought into line contact with a direction adjustment target. Since friction resistance received by a surface of the back surface support member 23 that came into contact with the direction adjustment target is reduced, the orientation around the central axis of the direction adjustment target can be easily changed while the back surface side of the direction adjustment target is supported.

The support surface 231 is preferably provided in a shape that enables the support surface 231 to be in point contact or line contact with a direction control target. The back surface support member 23 may be provided as a pad having a curved shape, a tube having a curved shape, or the like, or may be provided as a rotatable roller or the like.

The back surface support member 23 can be formed of an appropriate material. Examples of the material of the back surface support member 23 include elastomer, plastic, and metal. From the viewpoint of reducing impact on the direction control target, an elastomer such as rubber is preferably used as the material of the back surface support member 23.

Friction resistance received by the surface of the direction control mechanism 2 that came into contact with the protruding portion 102 is preferably larger than friction resistance received by the surface of the back surface support member 23 that came into contact with an installation object installed in the holder 110 which is a direction control target, for example, the body portion 101 of the blood collection tube 100 or the outer tube 200 that accommodates the blood collection tube 100. With such a friction resistance relationship, the orientation around the central axis of the direction adjustment target can be easily changed by the frictional force with the direction control mechanism 22 while supporting a back surface side of the direction adjustment target.

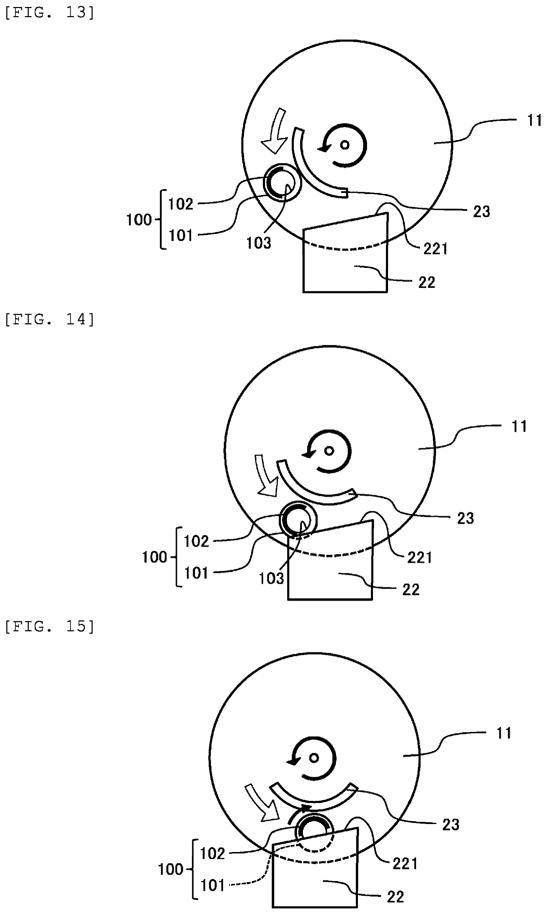

FIGS. 13, 14, and 15 are diagrams illustrating a method for mechanically adjusting the orientation around the central axis of the blood collection tube. FIGS. 13, 14, and 15 illustrate the periphery of the blood collection position illustrated in FIG. 11 as viewed from above. In these drawings, a detailed structure of the turntable 11, the holder 110, and the outer tube 200 are not illustrated.

FIGS. 13, 14, and 15 illustrate a process of adjusting a position of the protruding portion 102 relative to the central axis of the blood collection tube 100 by the mechanical direction adjustment mechanism 22. A target range is set in a half region located closer to the center side of the turntable 11 than the central axis of the blood collection tube 100. FIG. 13 illustrates a state where the blood collection tube 100 is moving toward a blood collection position. FIG. 14 illustrates a state where the blood collection tube 100 enters the blood collection position. FIG. 15 illustrates a state where the blood collection tube 100 is moved to the blood collection position.

As illustrated in FIG. 13, when the turntable 11 is controlled according to a blood collection operation or treatment, the blood collection tube 100 is moved toward the blood collection position on the turntable 11 with the rotation of the turntable 11. In a state where the blood collection tube 100 is moving toward the blood collection position, the orientation of the protruding portion 102 relative to the central axis of the blood collection tube 100 may be any orientation. Since an operator of the blood collection work can install the blood collection tube 100 in the holder 110 in any orientation, a burden such as checking the orientation is reduced.

As illustrated in FIG. 14, when the blood collection tube 100 comes in the order of blood collection, the blood collection tube 100 enters the blood collection position on the turntable 11 with the rotation of the turntable 11. The direction adjustment mechanism 22 having the guide surface 221 is installed at the blood collection position. When the position of the protruding portion 102 relative to the central axis of the blood collection tube 100 is out of the target range, the protruding portion 102 that is moving with the rotation of the turntable 11 abuts against the guide surface 221.

As illustrated in FIG. 15, when the blood collection tube 100 comes in the order of blood collection, the blood collection tube 100 is moved to and stopped at the blood collection position on the turntable 11 with the rotation of the turntable 11. The direction adjustment mechanism 22 is provided at a height at which the direction adjustment mechanism 22 can come into contact with the protruding portion 102 and at a height at which the direction adjustment mechanism 22 does not come into contact with the body portion 101 or the like. Therefore, while the body portion 101 is moved to the blood collection position with the rotation of the turntable 11, the movement of the protruding portion 102 along the circumferential direction of the turntable 11 is hindered by the guide surface 221.

When the position of the protruding portion 102 relative to the central axis of the blood collection tube 100 is out of the target range, the guide surface 221 comes into contact with the protruding portion 102 and moves the protruding portion 102 along the inclination. By moving the protruding portion 102 along the guide surface 221, the orientation around the central axis of the blood collection tube 100 is changed, and the position of the protruding portion 102 relative to the central axis of the blood collection tube 100 is guided within the target range.

On the other hand, when the position of the protruding portion 102 relative to the central axis of the blood collection tube 100 is within the target range, the guide surface 221 does not come into contact with the protruding portion 102 and allows the protruding portion 102 to move along the circumferential direction of the turntable 11. A gap through which the protruding portion 102 can pass is provided between the direction adjustment mechanism 22 and the back surface support member 23.

According to such a blood collection device, since the mechanical direction adjustment mechanism 22 is provided, the orientation around the central axis of the blood collection tube 100 can be changed using the rotation of the turntable 11 without using an actuator or the like. The position of the protruding portion 102 relative to the central axis of the blood collection tube 100 can be adjusted within a predetermined target range without introducing an electric device or additional control. In addition, it is not necessary for an operator who performs the blood collection work to check the orientation of the protruding portion 102, and the protruding portion 102 subjected to orientation adjustment can be used as a blood receiving surface or a receiving surface and a guide of an instrument. Therefore, the orientation around the central axis of the blood collection tube installed for blood collection can be adjusted with a simple device structure, work related to blood collection can be reduced, and efficient blood collection into the blood collection tube can be stably performed.

Although the embodiments of the invention have been described above, the invention is not limited to the embodiments described above, and various modifications can be made without departing from the gist of the invention. For example, the invention is not necessarily limited to one including all the configurations included in the embodiments described above. A part of a configuration of an embodiment may be replaced with another configuration, a part of the configuration of the embodiment may be added to another configuration, or a part of the configuration of the embodiment may be omitted.

### Reference Signs List

- 10:: housing
- 11:: turntable
- 12:: shaft
- 13:: rotation drive mechanism
- 13a:: motor
- 13b:: power transmission mechanism
- 14:: movable support member
- 15:: lifting drive mechanism
- 15a:: motor
- 15b:: power transmission mechanism
- 20:: position sensor
- 21:: direction adjustment mechanism
- 21a:: motor
- 21b:: direction adjustment unit
- 22:: direction adjustment mechanism
- 23:: back surface support member
- 100:: blood collection tube
- 101:: body portion
- 102:: protruding portion
- 103:: opening
- 110:: holder
- 120:: module
- 130:: cuff mechanism
- 131:: finger placement area
- 132:: finger placement component
- 133:: blood collection window
- 134:: finger of person from whom blood is to be collected
- 200:: outer tube

## Claims

1. A blood collection device comprising:
a holder configured to allow a blood collection tube to be installed;
a blood collection portion configured to collect blood from a person from whom blood is to be collected into the blood collection tube; and
a direction adjustment mechanism configured to adjust an orientation of the blood collection tube in the holder, wherein
the blood collection tube includes a bottomed cylindrical body portion having an opening in an upper portion, and a protruding portion formed by making a part of the opening in a circumferential direction protrude upward, and
the direction adjustment mechanism adjusts the orientation around a central axis of the blood collection tube such that a position of the protruding portion relative to the central axis of the blood collection tube falls within a predetermined range set in advance.

2. The blood collection device according to claim 1, further comprising:
a position sensor configured to detect a position of the protruding portion, wherein
the direction adjustment mechanism adjusts the orientation around the central axis of the blood collection tube based on a detection result of the position sensor.

3. The blood collection device according to claim 1, wherein
the direction adjustment mechanism includes a direction adjustment unit that comes into contact with an outer peripheral surface of the blood collection tube installed in the holder or an installation object that accommodates the blood collection tube, and a motor that operates the direction adjustment unit, and
the orientation around the central axis of the blood collection tube is adjusted by a frictional force caused by an operation of the direction adjustment unit.

4. The blood collection device according to claim 1, wherein
the direction adjustment mechanism includes a roller-type pad that comes into contact with an outer peripheral surface of the blood collection tube installed in the holder or an installation object that accommodates the blood collection tube, and a motor that rotates the pad, and
the orientation around the central axis of the blood collection tube is adjusted by a frictional force caused by the rotation of the pad.

5. The blood collection device according to claim 1, further comprising:
a turntable that is rotatably supported; and
a rotation drive mechanism configured to rotate the turntable, wherein
the holder is supported on the turntable.

6. The blood collection device according to claim 1, further comprising:
a turntable that is rotatably supported; and
a rotation drive mechanism configured to rotate the turntable, wherein
the holder is supported on the turntable,
the direction adjustment mechanism is a guide that is provided above the turntable at a height at which the guide comes into contact with the protruding portion and does not come into contact with the body portion, and
the orientation around the central axis of the blood collection tube is adjusted by moving the protruding portion along the guide with the rotation of the turntable.

7. The blood collection device according to claim 6, further comprising:
a support member on a side opposite to the direction adjustment mechanism across the protruding portion, the support member being configured to support the blood collection tube installed in the holder or an installation object that accommodates the blood collection tube from a side, wherein
the support member is provided above the turntable at a height at which the support member comes into contact with the body portion or the installation object that accommodates the blood collection tube and does not come into contact with the protruding portion.

8. The blood collection device according to claim 7, wherein
friction resistance received by a surface of the direction adjustment mechanism that comes into contact with the protruding portion is larger than friction resistance received by a surface of the support member that comes into contact with the body portion or the installation object that accommodates the blood collection tube.
